(19)

**Europäisches Patentamt**
**European Patent Office**
**Office européen des brevets**

(11) **EP 4 028 747 B1**

(12) **EUROPEAN PATENT SPECIFICATION**

(45) Date of publication and mention
of the grant of the patent:
**19.03.2025 Bulletin 2025/12**

(21) Application number: **20862110.2**

(22) Date of filing: **08.09.2020**

(51) International Patent Classification (IPC):
***G01N 11/06*** *(2006.01)*

(52) Cooperative Patent Classification (CPC):
**G01N 11/06**

(86) International application number:
**PCT/US2020/049689**

(87) International publication number:
**WO 2021/050418 (18.03.2021 Gazette 2021/11)**

(54) **METHOD AND APPARATUS FOR RHEOLOGY PROPERTY MEASUREMENTS OF DRILLING FLUIDS IN REAL-TIME**

VERFAHREN UND VORRICHTUNG ZUM MESSEN DER RHEOLOGISCHEN EIGENSCHAFTEN VON BOHRFLUIDEN IN ECHTZEIT

PROCÉDÉ ET APPAREIL DE MESURES DE PROPRIÉTÉS RHÉOLOGIQUES DE FLUIDES DE FORAGE EN TEMPS RÉEL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.09.2019 US 201962897662 P**

(43) Date of publication of application:
**20.07.2022 Bulletin 2022/29**

(73) Proprietor: **The Texas A&M University System College Station, TX 77843-3369 (US)**

(72) Inventors:
• **OFOCHE, Paul**
  **Bryan, TX 77801 (US)**

• **NOYNAERT, Samuel, F.**
  **College Station, TX 77845 (US)**

(74) Representative: **Whitfield, Gillian Janette**
  **Astrum ElementOne Limited**
  **Merlin House**
  **Langstone**
  **Newport NP18 2HJ (GB)**

(56) References cited:
**WO-A2-2018/175503      US-A- 5 437 194**
**US-A- 5 437 194           US-A1- 2004 069 069**
**US-A1- 2018 259 437    US-A1- 2019 178 770**
**US-A1- 2020 033 174    US-B2- 7 290 441**
**US-B2- 7 290 441**

**Description**

CROSS-REFERENCE TO RELATED APPLICATIONS

This application claims priority to U.S. Provisional Patent Application No. 62/897,662, filed on September 9, 2019.

TECHNICAL FIELD

The present disclosure relates generally to fluid measurements and more particularly, but not by way of limitation, to use of acoustic signals to obtain continuous real-time measurement of fluid rheological properties.

BACKGROUND

This section provides background information to facilitate a better understanding of the various aspects of the disclosure. It should be understood that the statements in this section of this document are to be read in this light, and not as admissions of prior art.

Drilling fluid plays an important role in rotary drilling wells. Serving as the means through which drill cuttings are lifted and downhole pressures controlled, the measurement and monitoring of drilling fluid properties helps improve the safety of operations. The drilling fluid density determines hydrostatic pressure while rheology (viscosity) affects the lifting capacity of the drilling fluid. A well-established method of measuring fluid rheology continuously has yet to be adopted by the petroleum industry. The present disclosure reveals the use of an acoustical technique to obtain real-time continuous measurements of fluid rheological properties and density.

Known systems and methods are disclosed in US5437194A, US7290441B2, US2019/178770A1, and WO2018/175503A2.

SUMMARY

This summary is provided to introduce a selection of concepts that are further described below in the Detailed Description.

Aspects of the disclosure relate to a system for monitoring fluid properties in real-time according to independent claim 1.

In some embodiments, the system further includes a wave generator electrically coupled to the sensors. In some embodiments, the fluid flow is maintained at a continuous and constant volume flow. In some embodiments, the sensors utilizes acoustic signals to non-intrusively measure fluid properties.

Aspects of the disclosure relate to a method for monitoring fluid properties in real-time according to independent claim 9.

BRIEF DESCRIPTION OF THE DRAWINGS

A more complete understanding of the subject matter of the present disclosure may be obtained by reference to the following Detailed Description when taken in conjunction with the accompanying Drawings wherein:

FIG. 1 illustrates an acoustic measurement device according to an aspect of the present disclosure.

FIG. 2A illustrates the variance of resonance frequency with shear rate using $5.11s^{-1}$ piezo discs.

FIG. 2B illustrates the variance of resonance frequency with shear rate using $10.21s^{-1}$ piezo discs.

FIG. 2C illustrates the variance of resonance frequency with shear rate using $170.23s^{-1}$ piezo discs.

FIG. 2D illustrates the variance of resonance frequency with shear rate using $340.46s^{-1}$ piezo discs.

FIG. 2E illustrates the variance of resonance frequency with shear rate using $510.69s^{-1}$ piezo discs.

FIG. 2F illustrates the variance of resonance frequency with shear rate using $1021.38s^{-1}$ piezo discs..

FIG. 3A illustrates compared viscosity and density readings of methylcellulose solution for a range of shear rates with those obtained from a conventional rotational viscometer and a mud scale.

FIG. 3B illustrates compared viscosity and density readings of guar gum for a range of shear rates with those obtained from a conventional rotational viscometer and a mud scale.

FIG. 4A shows the shear-thinning tendency displayed by a partially hydrolyzed polyacrylamide (PHPA) solution.

FIG. 4B shows the shear-thinning tendency displayed by a calcium chloride ($CaCl_2$) solution.

FIG. 5 illustrates compared viscosity and density readings of glycerol-water solution for a range of shear rates with those obtained from a conventional rotational viscometer and a mud scale. Despite being a Newtonian fluid, predicted densities are mostly precise and viscosity readings from the acoustic technique are reasonably accurate within $\pm 2$ cP across all sensors.

FIG. 6 illustrates a telescopic six-diameter flow loop according to an aspect of the present disclosure.

FIGS. 7A-7D illustrate tests on four concentrations of calcium chloride ($CaCl_2$) solution (with densities ranging from 9.05 to 11.5 lb/gal) showing close matches between the acoustic technique when comparing viscosity and density results with those obtained from a conventional rotational viscometer and the mud balance.

FIGS. 8A-8D illustrates tests on four liquids/muds showing a mostly close match between the acoustic technique when comparing viscosity and density results with those obtained from a conventional rotational viscometer and the mud balance.

FIG. 9 illustrates a close match when comparing viscosity and density results from the acoustic flow loop with those obtained from a conventional rotational viscometer and the mud balance on an Eagle Ford (Texas) drilling mud.

FIG. 10 is a flow diagram illustrating a method for acoustic measurement of fluids according to aspects of the disclosure.

## DETAILED DESCRIPTION

In the following detailed description some non-SI, cgs, units are used, which convert into SI units as follows:

viscosity: 1 centipoise (cP) = 0.01 Poise (P)
density: 1 g/cm$^3$ = 1000 kg/m$^3$

It is to be understood that the following disclosure provides many different embodiments, or examples, for implementing different features of various embodiments. Specific examples of components and arrangements are described below to simplify the disclosure. These are, of course, merely examples and are not intended to be limiting. The section headings used herein are for organizational purposes and are not to be construed as limiting the subject matter described.

According to aspects of the disclosure, a pair of sensors is used to transmit and record the behavior of waveforms passed through the fluid of interest. The sensors are piezoelectric transducers. Liquids with known densities and viscosities are sampled in a customized three-dimensional printed flow loop and used to characterize the system and build a predictive model. Some modeling work is done to compare the conformance of the examples with established physics concepts. Statistical and machine-learning concepts are applied to analyze and interpret the results.

Miscellaneous unknown properties or compositions are tested in the flow loop and the signals are analyzed to deduce rheological properties. The ability to continuously sweep the generated waveforms over a range of frequencies means that the data collection can be automated and used to achieve real-time surveillance of the fluid properties.

Working Examples

Reference will now be made to more specific embodiments of the present disclosure and data that provides support for such embodiments. However, it should be noted that the disclosure below is for illustrative purposes only and is not intended to limit the scope of the claimed subject matter in any way.

Measuring Fluid Rheology and Density in Real-Time Using Sound Signal

Well known methods exist for the measurement of density under fluid flow. Likewise, significant effort has been put into developing means for measuring rheology. Rheology is the study of how matter deforms. Liquids deform continuously when stress is applied. They are classified as either Newtonian (viscosity independent of shear rate) or non-Newtonian (viscosity dependent on shear rate). The vast majority of fluids exhibit non-Newtonian tendencies, meaning their viscosities cannot be expressed by a single number and need to be measured under varying flow conditions/shear rates. In either type of fluid, the viscosity of fluids is dependent on its temperature, thus it must be expressed in relation to a specified temperature. These factors help explain the complexities associated with rheology quantification and why the expression of viscosity is less intuitive than density.

A wide range of viscometers exist to measure viscosity, and can include, for example, capillary viscometers, rotational viscometers, moving body viscometers, and the like. To control all factors associated with rheological measurement and to standardize the measurement of viscosity, most viscometers are designed to operate under set conditions. This means that the fluids are not measured at in situ conditions. Many attempts have been made at designing in-line viscometers for real-time viscosity measurement. The resulting commercially available methods are often expensive and possess moving parts which limit their robustness and adaptability to different flow systems.

The present disclosure provides an approach to measure both viscosity and density of fluids under flowing conditions using an acoustic technique. Density concerns the amount of matter contained in a unit volume of a substance; whereas (dynamic) viscosity is the shear stress required to drag a layer of fluid with unit velocity past another layer a unit distance away from it in the fluid. Molecular interchange and cohesive forces between molecules are responsible for viscosity in liquids. The present disclosure shows that density and viscosity can be measured accurately by the propagation and damping of sound signals and the resonance phenomenon. Real-time measurements of both properties help improve operational performance in fluid systems. The results obtained using the new acoustic method was compared with measurements from a conventional concentric cylinder viscometer. Propagation of sound signals in fluid media was also compared with simulations from a computer model to help portray the underlying mechanism of operation.

The acoustic method for simultaneously measuring density and viscosity is focused on the phenomenon of damping that occurs when sound waves pass through fluid media. A waveform generator creates signals which are emitted into the fluid sample by a piezoelectric disc, swept repeatedly over a range of frequencies and captured by an identical receiver piezo disc. The dampened sound signals cause a deflection of the piezoelectric receiver that converts the mechanical energy into electrical voltage recorded by a data acquisition (DAQ) device for analysis and interpretation on a computer.

Beginning with air and pure liquids of known properties, the acoustic apparatus is calibrated and characterized to a baseline reference equivalent to conventional units by use of statistical tools. The system is then used to measure unknown fluids. The resonance frequency is seen to have an inverse relation with density, whereas higher viscosities result in a reduction of the voltage amplitude (damping) as well as a broader peak at resonance (widened bandwidth). The ability to generate the signals and record the response repeatedly means that the fluid properties can be monitored continuously. Thus, real-time fluid surveillance is achieved in an automated manner without the need for sample collection. This acoustic technique can also be applied to a wider range of viscosities than some viscometers can operate on. The density and viscosity of the fluids are be visualized and analyzed to obtain results in real-time. The set-up, as disclosed herein, is inexpensive and easy to mount on existing facilities.

The measurements can be performed both for static fluids and during circulation. The equipment utilized for measuring the density and rheology of fluids include two piezoelectric transducers, a conduit for conveying the fluid tested, an arbitrary waveform generator, a data acquisition (DAQ) unit, and a computer for displaying and analyzing the data. In order to study the shearing effects of flow rates and temperature, other components such as a pump and heating device (with thermometry), as well as a flow loop may be incorporated. Unlike the popular Time of Flight ultrasonic flowmeters used to measure flow velocity which operate based on the time lag of sound pulses between a transmitter and receiver, the transceivers of the present disclosure are arranged parallel to each other and perpendicular to the flow direction, hence there is no Doppler Effect (Doppler shift frequency) arising from the flow.

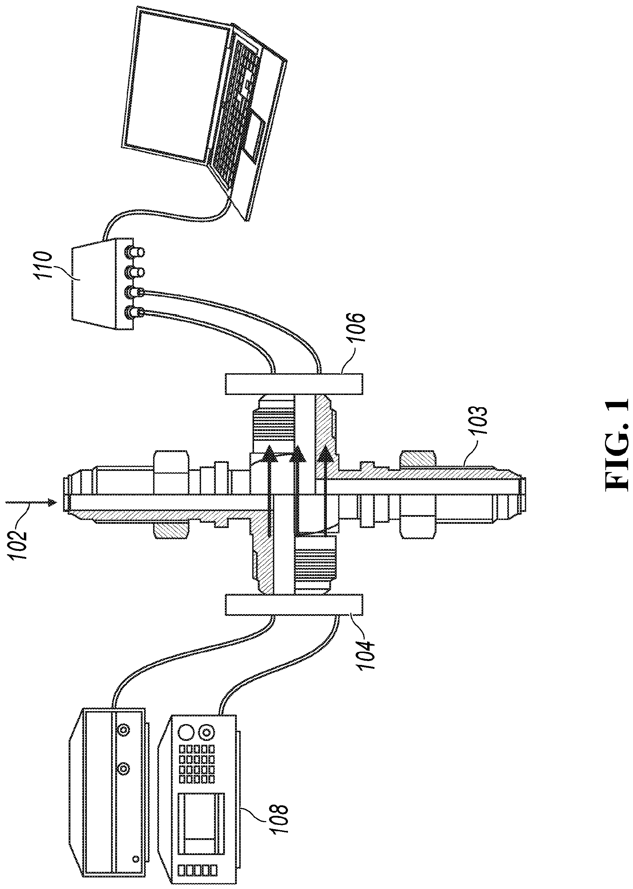

FIG. 1 illustrates an acoustic measurement device 100. The fluid 102 to be measured is placed in a conduit 103 between a first sensor 104 and a second sensor 106. In various embodiments, the first sensor 104 is a first piezoelectric disc and the second sensor 106 is a second piezoelectric disc. The first sensor 104 serves as a transmitting source, while the second sensor 106 is used as a receiver to record the effect of the effect of a wave that is passed through the fluid 102. A waveform generator 108 produces an electrical waveform propagated from the first sensor 104, which is swept through a pre-selected frequency range. The second sensor 106 converts the dampened wave signals into a small voltage output by oscillating due to excitation. The data acquisition unit ("DAQ") 110 records a frequency response and a voltage generated by the second sensor 106 by use of a fast Fourier transform (FFT) routine. Alternatively, for quicker results this example can be conducted in the pulsing mode, whereby the first sensor 104 is used to produce multiple pulses that are propagated through the fluid 102 and captured by the second sensor 106. The FFT algorithm is used to convert the signals from time to frequency domain for further analysis to deduce characteristics of the fluid.

The selection of the first sensor 104 and the second sensor 106, as used herein, is based upon resonance. Additionally, to incorporate the interactions of various trends observed in the acquired data, machine-learning techniques are employed to interpret the data. This differs from current practices used for analysis of fluid properties. The machine-learning algorithms harness the impacts of key trends in the data to attain a high level of measurement accuracy. These trends include, but are not limited to, resonant frequency, kurtosis, quality factor, and correlation coefficient. Ensemble learning models using the various factors, such as, without limitation, kurtosis, and quality factor were developed for prediction of the sensors' response. Robustness and the improved accuracy of results are benefits derived from this approach. A mud balance and a viscometer were used to measure and calibrate for the density and viscosity, respectively, of dozens of fluid samples of varying properties. Results from this calibration set were used as the training samples in building models for the system response.

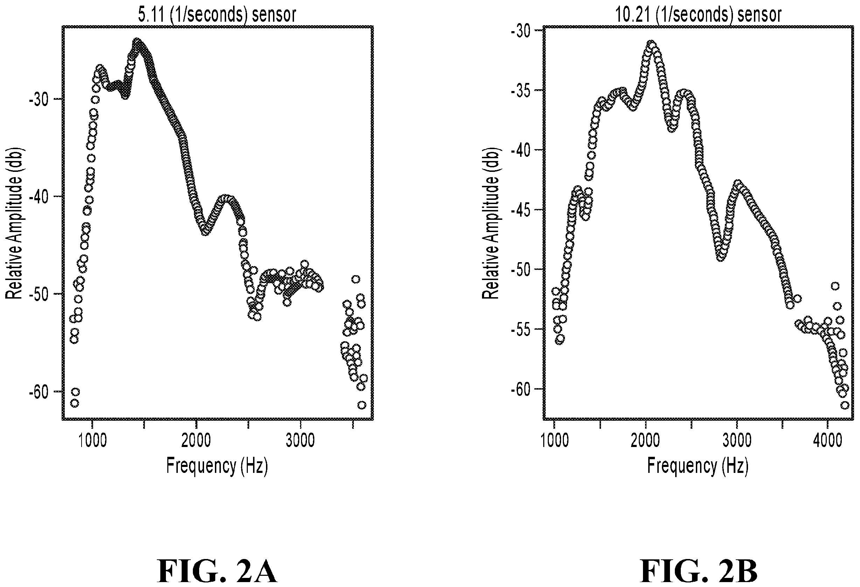

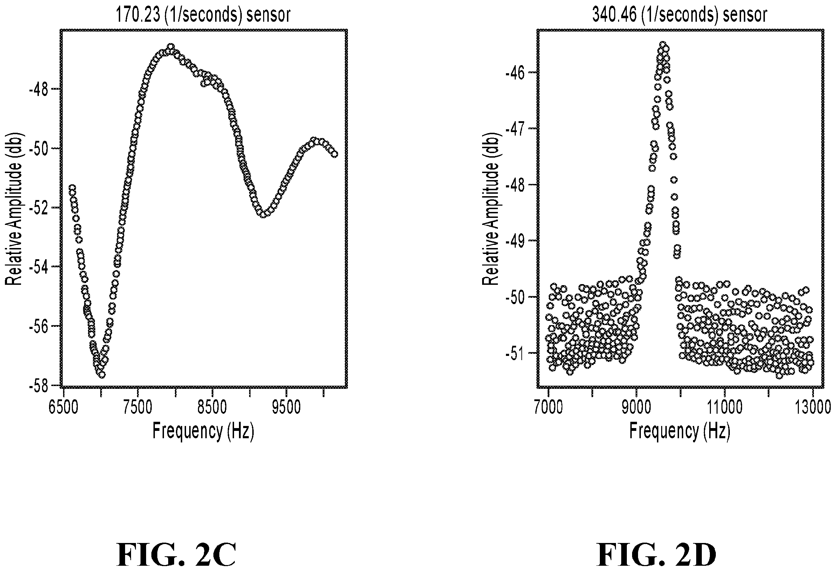

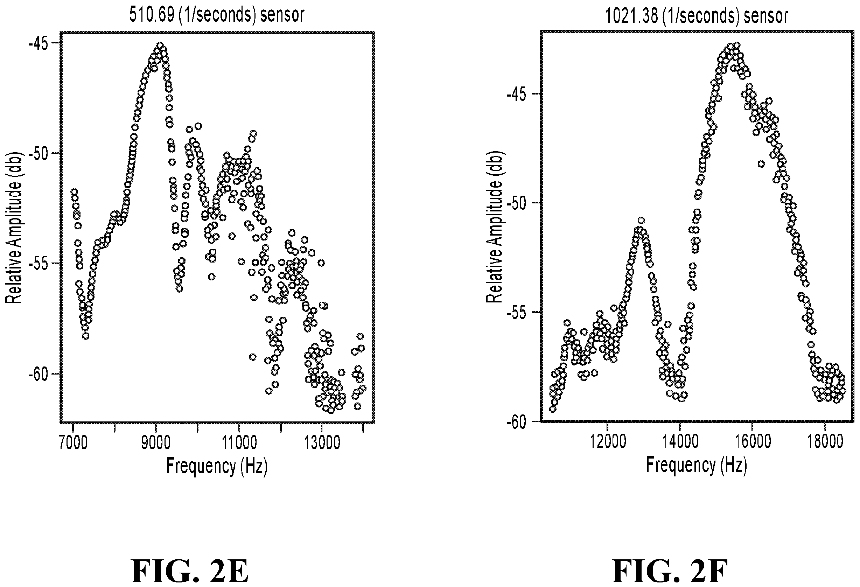

Various fluids were tested utilizing the acoustic measurement device 100 and the viscosity and density results were compared with those from a conventional viscometer and mud balance. The typical signals recorded from each of the six sensor pairs are shown in FIGS. 2A-2F. FIG. 2A illustrates the variance of resonance frequency with shear rate using 5.11s$^{-1}$ sensors. FIG. 2B illustrates the variance of resonance frequency with shear rate using 10.21s$^{-1}$ sensors. FIG. 2C illustrates the variance of resonance frequency with shear rate using 170.23s$^{-1}$ sensors. FIG. 2D illustrates the variance of resonance frequency with shear rate using 340.46s$^{-1}$ sensors. FIG. 2E illustrates the variance of resonance frequency with shear rate using 510.69s$^{-1}$ sensors. FIG. 2F illustrates the variance of resonance frequency with shear rate using 1021.38s$^{-1}$ sensors. It can be observed that the resonance frequency increases with increased shear rate as the sensor size is reduced. The 5.11 s$^{-1}$ sensor pair was the largest, with a diameter of 65 millimeters and the greatest amount of surface exposed to fluid interaction, whereas the 1021.38 s$^{-1}$ sensor pair was the smallest (27 mm) and had the least exposed surface. After generating the waveforms and sweeping them over a range of frequencies, the signals are then fed to interpretative algorithms for analysis and a report of the density and rheological properties are deduced.

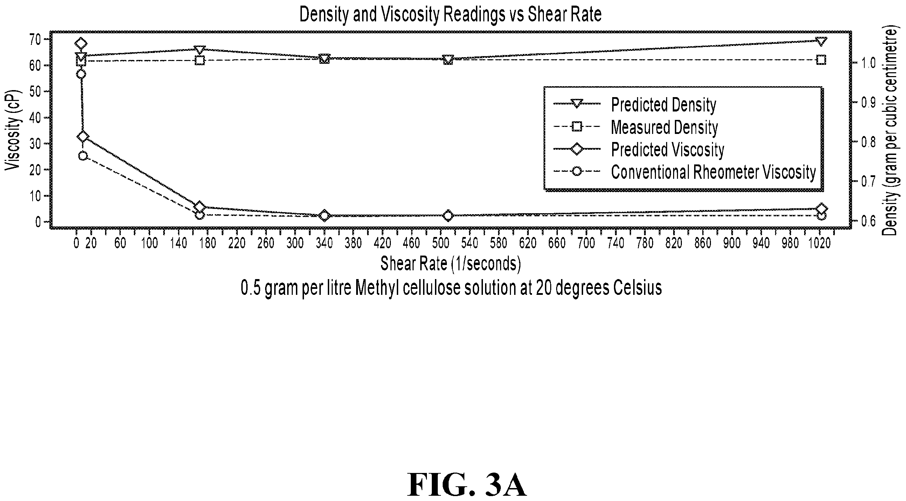

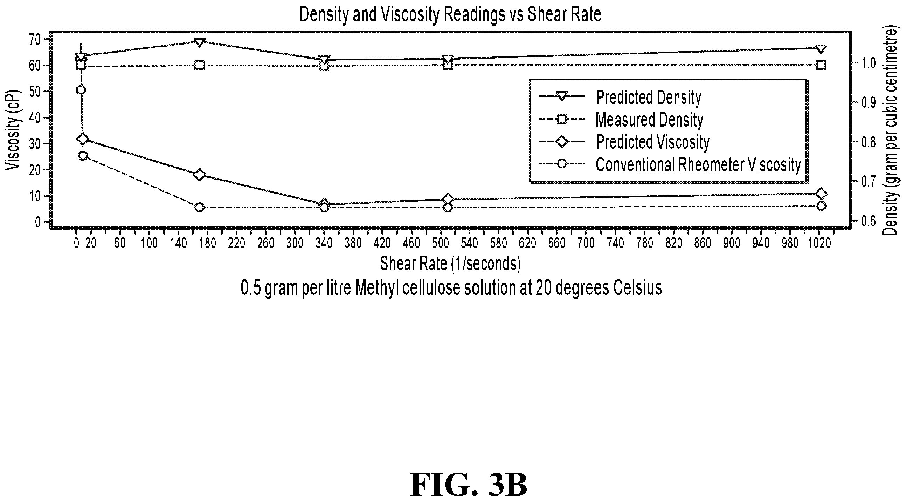

The model was used to predict the density and viscosity on test samples having both Newtonian and non-Newtonian fluids. The shear thinning effects of methylcellulose solutions is illustrated in FIG. 3A and the shear thinning effects of guar gum solutions is illustrated in FIG. 3B. These non-Newtonian viscosifiers are known to exhibit thixotropic behaviors with their viscosities decreasing markedly with constant shear over time. With mud scale measured densities of 1.0065 and 0.998 g/cm$^3$, respectively, for the methylcellulose and guar gum solutions and assuming homogenous dispersion across all sensors, a close match is obtained with the densities measured using the acoustic technique. The largest discrepancies in density measurements are observed on the 170.23 s$^{-1}$ and the 1021.38 s$^{-1}$ sensors for tests on both fluids. For the viscosity measurements, the predictions at the higher shear rate sensors are generally accurate while moderate discrepancies are observed on the 5.11 and 10.21 s$^{-1}$ sensors with the exception of the 170.23 s$^{-1}$ sensor which shows the widest deviation on the guar gum test. The thixotropic or shear thinning effects of these viscosifiers are obvious and well captured by the acoustic measurements with viscosities declining from about 60 cP to 1 cP for the methylcellulose solution and from about 20 cP to 2 cP for the guar gum solution in the range of shear rates applied.

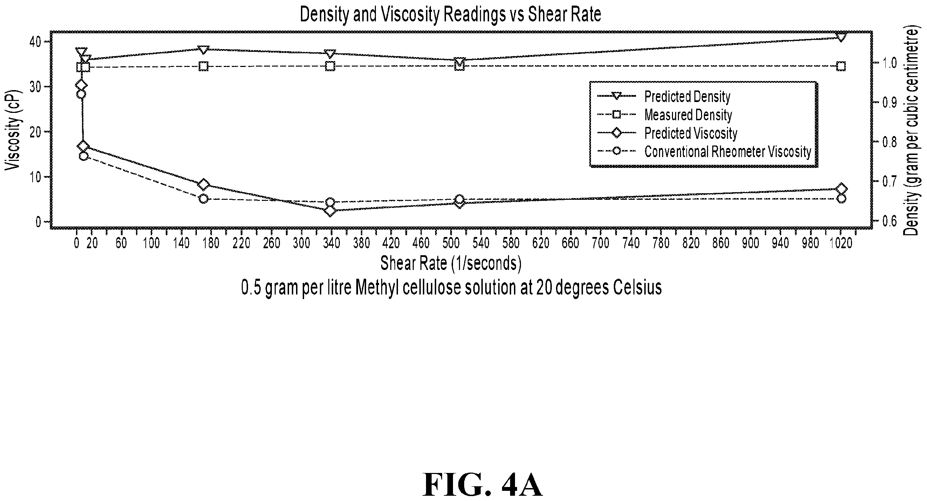

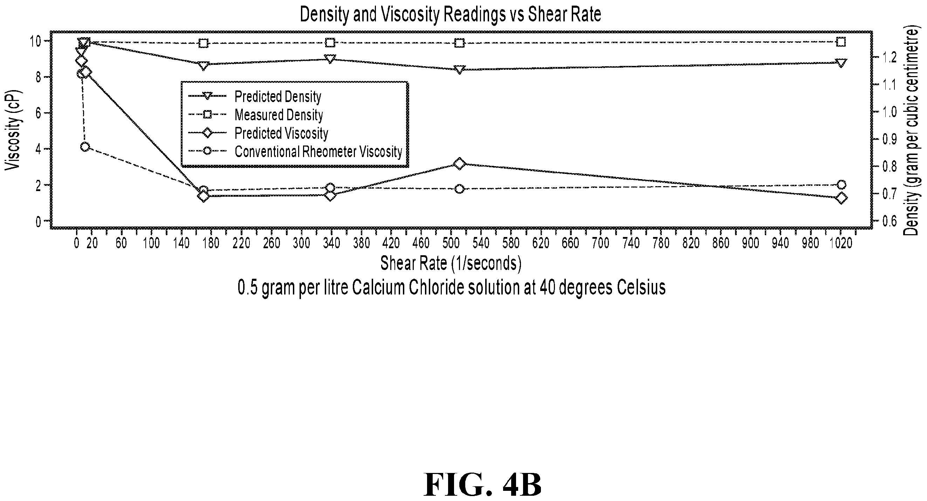

FIG. 4A shows the shear-thinning tendency displayed by a partially hydrolyzed polyacrylamide (PHPA) solution and FIG. 4B shows the shear-thinning tendency displayed by a calcium chloride (CaCl$_2$) solution. From about 30 cP down to 7 cP, and from about 9 cP down to 2 cP for PHPA and CaCl$_2$, respectively, the fluids thin out at increased shear rates. The ranges of shear are a bit different, but the precision of detection using the acoustic technique is clearly seen. PHPA is a polymer that is well known in the drilling industry and sometimes used as a viscosifying agent in mud. CaCl$_2$ is used amongst other things to increase the density of muds in the oil industry with densities ranging from 1 to 1.39 g/cm$^3$ at saturation. The particular concentration of CaCl$_2$ used for the test in FIGS. 4A-4B had a density of 1.25 g/cm$^3$ which was under-predicted most of the sensors; on the other hand, the density of the PHPA solution was over-predicted for most of the sensors. The maximum discrepancies across both fluids were at ±0.076 g/cm$^3$. A number of factors could be attributed to these, such as the unproven assumption of complete homogeneity, as well as the limited entrance length of 9 inches across all the sensors which did not suffice for a fully developed laminar flow while generating the shear.

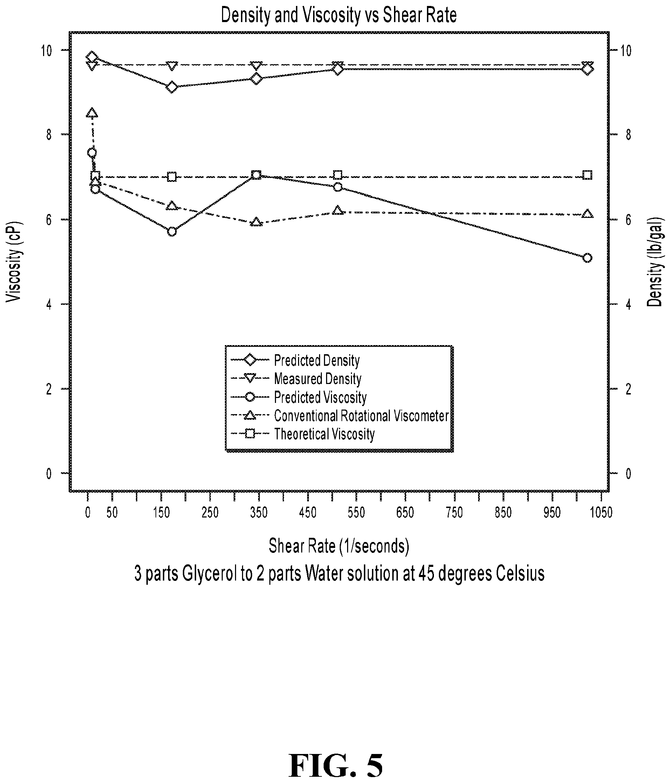

Vibrational viscometers are utilized to measure non-Newtonian fluids, but less accurate with Newtonian fluids. Since the acoustic method discussed herein is considered a vibration type viscometer, a Newtonian fluid such as a glycerol-water mixture was used, given that the prediction model was also trained on mostly non-Newtonian fluids. Furthermore, FIG. 5 shows results for a glycerol-water solution and a close match was obtained in comparison with measurements from a mud balance and a conventional rotational viscometer.

The glycerol-water solution is a Newtonian fluid so, theoretically, the viscosity is expected to remain fairly constant across the various shear rates (i.e. no shear thinning). The proportion of the solution was 3 parts glycerol and 2 parts water and the example was performed at 45 °C (104 °F), so there is a well-documented expectation of the density and rheological properties. The viscosity should be 7 cP across all shear rates and the density is 9.65 lb/gal (1.16 g/cm$^3$). It can be seen that density predictions closely matched those recorded on the mud balance and stated in literature. The viscosity detected acoustically had some deviations from the theoretical value, but so do conventional rheometer.

As demonstrated in the present disclosure, the acoustic method presented herein for measuring viscosity and density of fluids in real-time based on resonance phenomenon. The shearing thinning effects of the non-Newtonian fluids demonstrated herein are adequately captured across a wide range of shear rates which represent the applications in which the acoustic methods would be considered for use. Statistical principles and machine-learning concepts were employed to develop a model using the Multivariate Random Forest method for accurately interpreting and predicting the properties of unknown fluid samples. The analytical model improves with a loop of data. Further tests on different fluid types can widen the training sets and improve the models' predictive performances. The techniques disclosed herein are useful in various industries requiring continuous monitoring of fluid properties under flowing conditions. Based on the results from the many tests carried out with the acoustic viscometer, the techniques are suitable to adapt to industrial applications to fully utilize its potentials.

## Equivalence of Conventional Rheometer Readings with Results from an Acoustic Method

The common reference for the procedures used for field-testing of water-based drilling fluids is the American Petroleum Institute's (API's) recommended practice document. Some of the main properties that describe the quality of a drilling fluid include, for example, the density, viscosity, and gel strength. The standard instruments used in the oil industry to measure density and viscosity are a mud balance and a Marsh funnel, respectively. A more sophisticated device, the concentric cylinder viscometer, is also used to measure viscosity and gel strength. Testing procedures however require that samples be taken and analyzed to obtain the desired readings. For rheology determination, the mud sample is measured at six rotational frequencies: 3, 6, 100, 200, 300, and 600 revolutions per minute (rpm). The readings are used to characterize the qualities of the drilling fluid under a variety of operational conditions. Since they are periodic measurements, once every 12 to 24 hours in many cases, they are not adequate for the present day's quest for automation of drilling activities. Thus, attention has steadily been given to methods for improving the rate of mud property data collection with the least possible human interaction.

Information about the mud and drilling process in general can be derived from rheological parameters. Owing to this, it follows that a more rapid and precise measurement of the fluid's properties will further enhance drilling performance initiatives around the industry.

The present disclosure introduces another means of obtaining real-time density and rheology data by use of sound signals generated by a piezoelectric transducer, swept through the fluid of interest and detected by an identical receiver piezo transducer. In keeping with the recommended practices detailed in API documentation, the drilling fluid is sampled at the six aforementioned standard shear rates and the results are simultaneously analyzed to derive the viscosity under a range of operational conditions. Automated drilling fluid sample tests are among the procedures that can contribute to safer and improved Managed Pressure Drilling (MPD) operations.

The acoustic approach employed in the present disclosure gives additional capabilities, such as: (1) continuous, concurrent measurement of rheology at the six API standard shear rates; (2) a wider range of measurement even for highly viscous fluids that exceed the scale of some rotational viscometers; (3) precise detection of slight changes in mud density and viscosity, as well as progressive gelation trends; and (4) estimation of the gas content in drilling fluids.

The viscosity of a fluid is defined as a measure of its resistance to gradual deformation by shear or tensile stress. In the petroleum industry, most conventional viscometers (also known as rheometers if they can measure viscosities at varying flow conditions) are of the rotational type. These concentric cylinder-type rheometers are either Couette-type (outer rotating cylinder) or Searle-type whereby the inner cylinder rotates. Rotational viscometers can also be classified into two categories based on the driving mechanism of the main shafts as either synchronous stepper motor spring or the digital encoder servo motor. The viscosity of the sample is captured by the spring deflection or the current required to drive the rotor at the particular testing speed.

The present disclosure presents a different type of rheometer used, in which flow is through a pipe as described by the Poiseuille flow. Sound waves generated by a piezoelectric transducer are passed though the fluid and the resultant damping effect of the signals are used to drive a receiver sensor. The voltage pattern generated by the receiver is recorded by a data acquisition (DAQ) device and interpreted on a computer. In the present disclosure, the Poiseuille flow methodology is adopted instead of the Couette flow approach utilized in the operational design of most rotational viscometers. Poiseuille flow models pipe flow which represents real world application and allows for a better measurement device among other things.

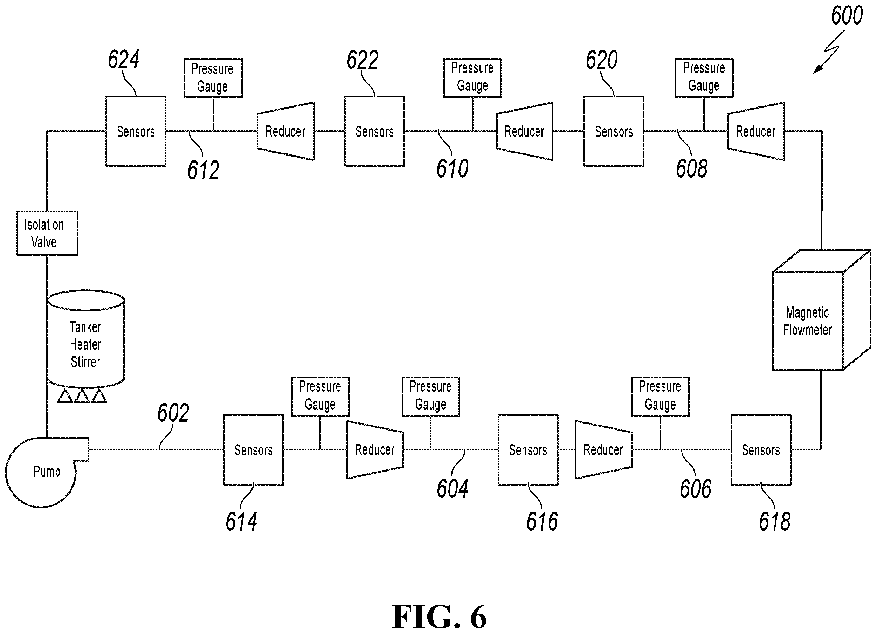

FIG. 6 depicts a constant rate flow loop 600 designed to measure the six API recommended shear rates simultaneously. The flow loop 600 is based on the simple shear calculated for equivalent Couette flow corresponding to a viscometer design. For a Newtonian fluid, the inner pipe wall shear rate ($\gamma$) for a flowing fluid is obtained by Equation 1, shown below, where d is the inner diameter of the pipe section.

**Equation 1**

$$\gamma = \frac{4Q}{\pi \cdot r^3} = \frac{32Q}{\pi \cdot d^3}$$

To replicate the six shear rates stipulated in API documentation, six corresponding pipe sections (602, 604, 606, 608, 610, 612) with six different pipe diameters were calculated based on a constant flow rate of 1 gallon per minute as shown in Table 1. Table 1, shown below, illustrates flow loop dimensions with corresponding shear rates with a 1 gallon per minute flow rate.

**Table 1**

| Section | Q (gpm) | Q (cm³/s) | Q (liter/s) | d (inch) | d (cm) | $\Upsilon$ (s⁻¹) |
|---------|---------|-----------|-------------|----------|--------|----------|
| 602 | 1 | 63.0902 | 0.06309 | 1.97 | 5.0038 | 5.129348 |
| 604 | 1 | 63.0902 | 0.06309 | 1.565 | 3.9751 | 10.23099 |
| 606 | 1 | 63.0902 | 0.06309 | 0.613 | 1.5570 | 170.247 |
| 608 | 1 | 63.0902 | 0.06309 | 0.4865 | 1.2357 | 340.5746 |
| 610 | 1 | 63.0902 | 0.06309 | 0.425 | 1.0795 | 510.8508 |
| 612 | 1 | 63.0902 | 0.06309 | 0.3374 | 0.8569 | 1021 |

To obtain the precise inner diameters listed in Table 1 above, three-dimensional (3D) printing is used to create each pipe section to fit the desired specification. Six sensor pairs (614, 616, 618, 620, 622, 624) are placed at each pipe section to measure the fluid properties at the six shear rates throughout the entire flow line 600.

Sensors are situated 9 inches from the entrance of each pipe section. Hence, with an entrance length of 9 inches, the maximum permissible Reynolds Numbers to maintain fully developed laminar flow in the widest and narrowest sections of the flow loop are 76 and 445. This is quite a low Reynolds number given that the laminar flow limit occurs at 2100. It will require low flow rates or highly viscous fluids to achieve fully developed laminar flow all across the flow loop. The operational design for this example has a constant flow rate of 1 gpm, meaning that only highly viscous fluids can attain these low Reynolds numbers. The laminar flow condition is a preferred condition in fluid mechanics since it is predictable and accurately definable with mathematical equations, unlike turbulent flows which are at best roughly approximated in simulations. Nevertheless, the findings in this example were successfully conducted in non-laminar flow conditions indicating the proposed model is robust and ready for applications in all flow types.

In order to create a process that was API compliant, the flow loop 600 was constructed to have six sections to achieve shear rates equivalent to that obtained with a viscometer. Having established the modes of operation and expected acoustic signal trends next was to adapt the technique to perform mud tests. An ensemble method such as the Multivariate Random Forest algorithm is used to train and build predictive models on each of the six pairs of sensors with corresponding data obtained from density and viscosity calibration measurements made by the viscometer. Multiple output linear regression models were initially adopted and a backward elimination approach was used to remove insignificant variables after initially including all parameters into the model. While this approach gave results that indicated it was the correct overall process, the best-adjusted R-squared values achieved were around 40%. Therefore more sophisticated non-linear tree-based models were utilized.

Viscosity is naturally a more difficult property to measure than density. Unlike density which requires only two dimensions (mass and length), viscosity is defined by three dimensions (mass, length, and time) and the quantity is dependent on temperature. Even water has a temperature-dependent viscosity, where the standard 1 cP viscosity commonly used only occurs at approximately 20 °C (68 °F). To emphasize this further, it is possible to calculate the density of mixtures using well-defined relationships, whereas that is not the case for viscosity. The best of conventional rotational viscometers specify that readings at low shear rates are questionable and it is necessary to perform some trials to determine the correct bob and spring combinations to use for various types of fluids under a range of test conditions. To counter the variabilities of measurement that come with certain ranges of viscosities, other types of viscometer are used. Various types of viscometers, such as, for example, falling ball or cone and plate, are used in some applications. Despite these limitations to the rotational viscometer, it is still considered the standard for API fluid measurement. Thus for this example, comparisons are made to results from a conventional rotational viscometer.

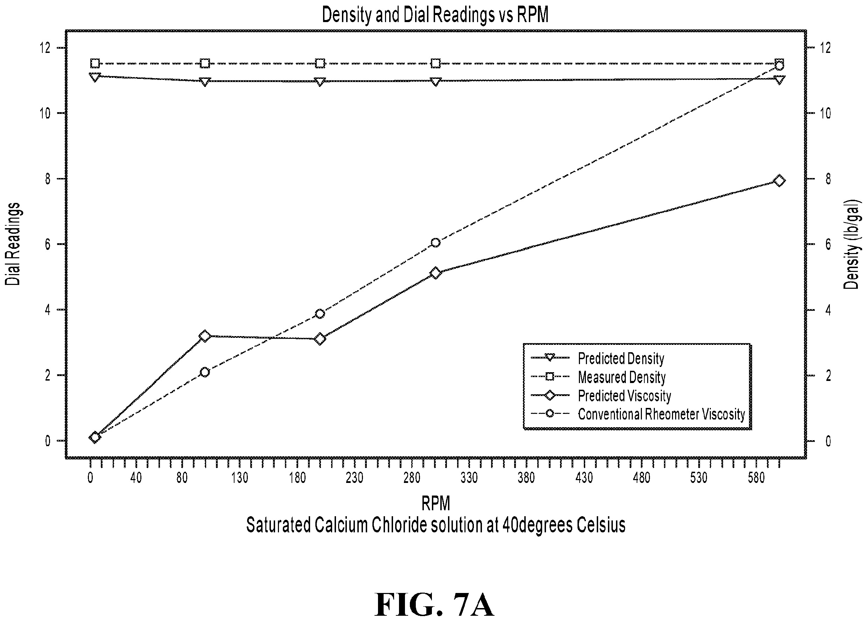

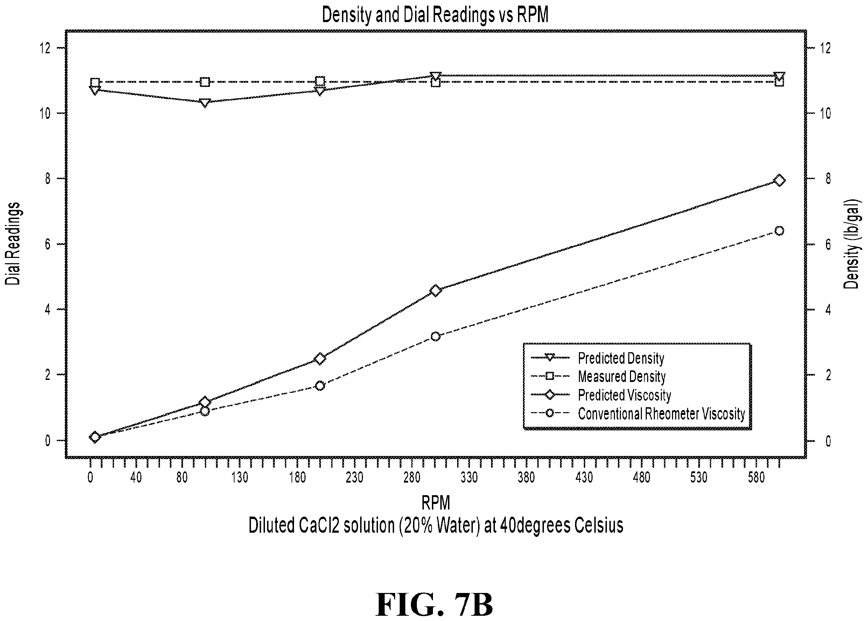

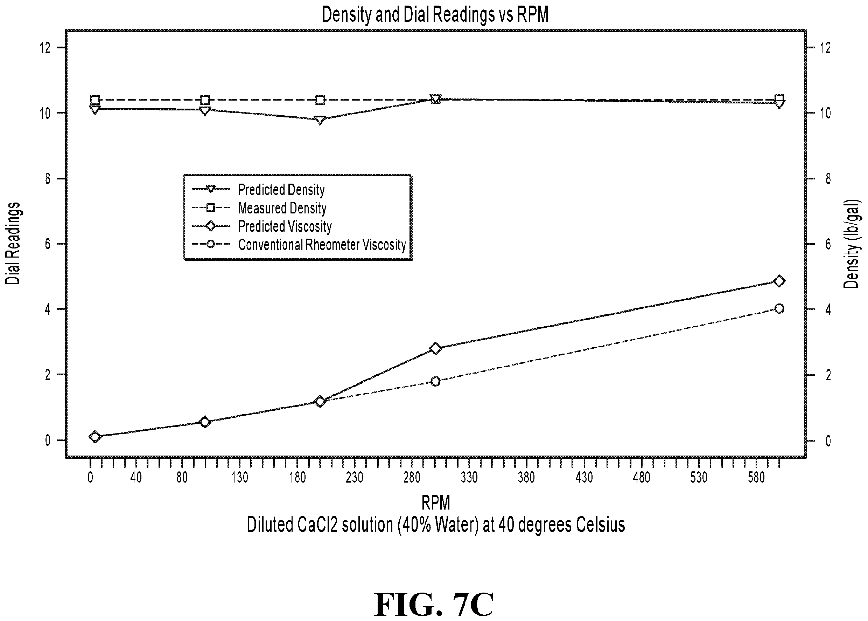

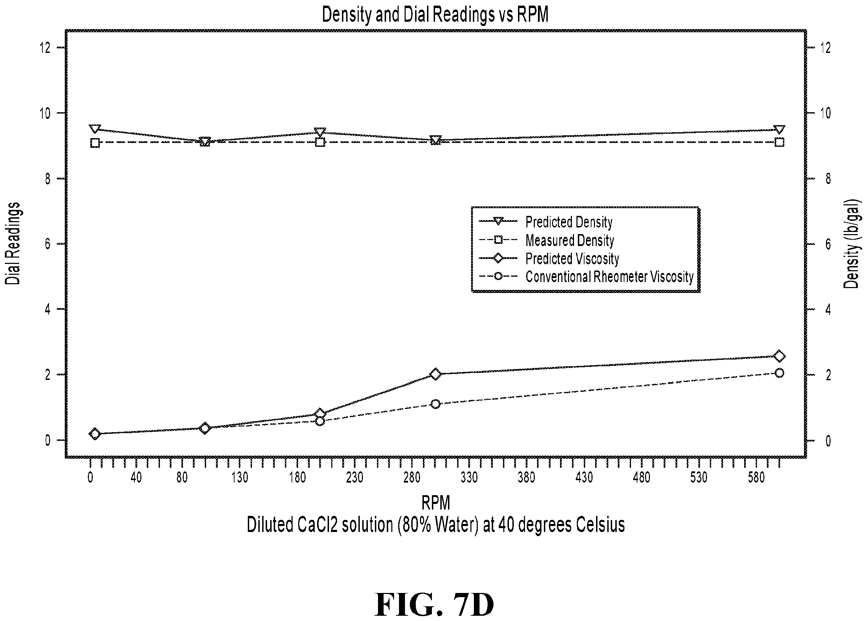

Tests were conducted on a variety of fluids to determine the accuracy and robustness of the acoustic viscometer. All tests were performed at 40°C (104°F). Four different concentrations of calcium chloride ($CaCl_2$) solution were pumped through the flow loop and the recorded real-time results across the six sensors are presented in FIGS. 7A-7D. FIG. 7A illustrates saturated calcium chloride solution. FIG. 7B illustrates calcium chloride solution diluted with 20% water. FIG. 7C illustrates calcium chloride solution diluted with 40% water. FIG. 7D illustrates calcium chloride solution diluted with 80% water. Saline solutions are used, as they are homogenous solids-free fluids and expected to be a check on the efficacy of the sensing technique. Expectedly, the density and viscosity variations among the fluids and at the sections of the loop are observed to match values measured by the conventional rheometer and the mud balance. Despite the pipe lengths at each section (9 inches) falling well short of the required entrance length for attaining fully developed flow, the results still show good accuracy.

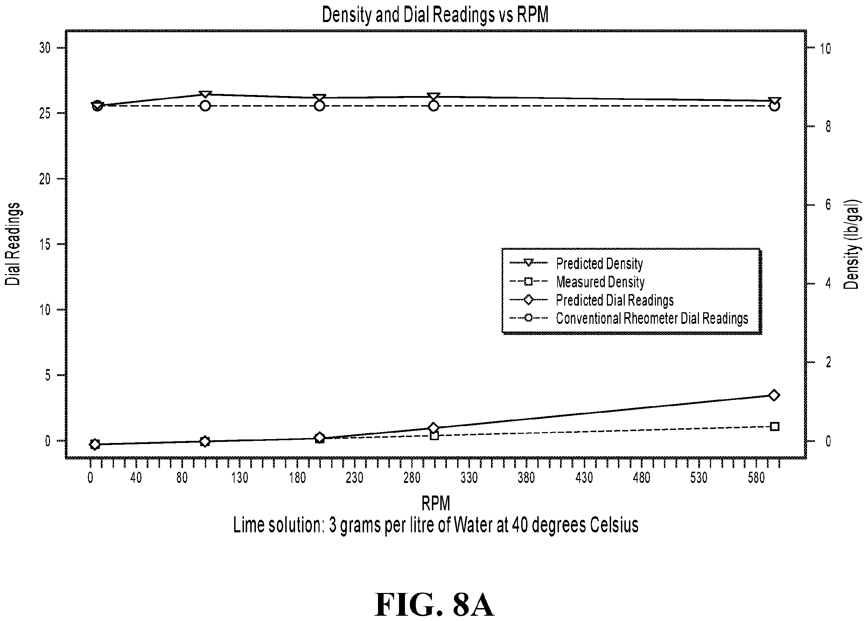

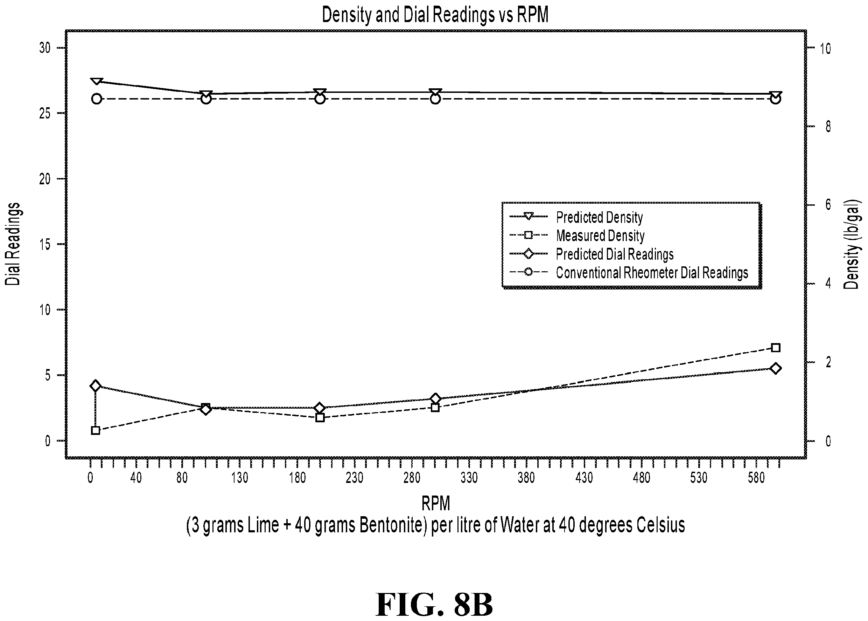

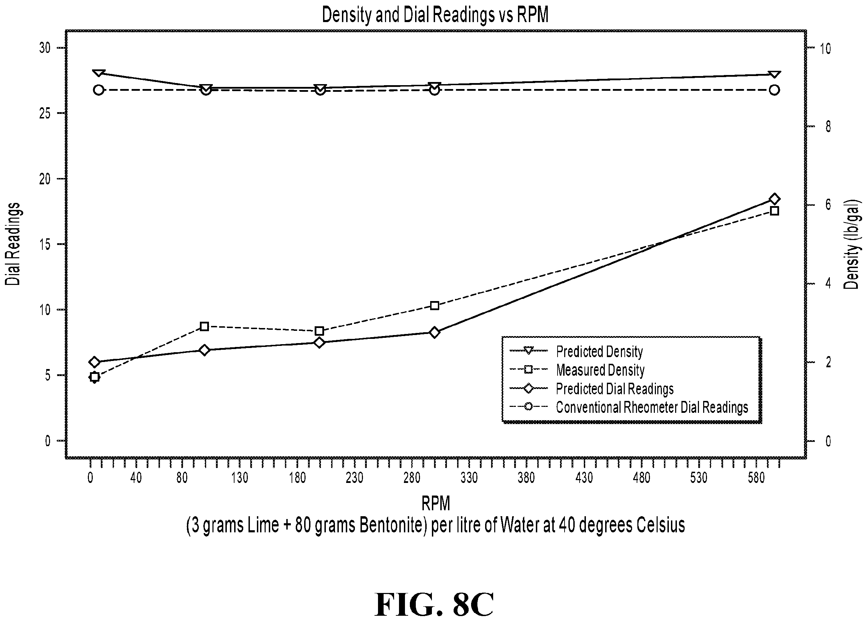

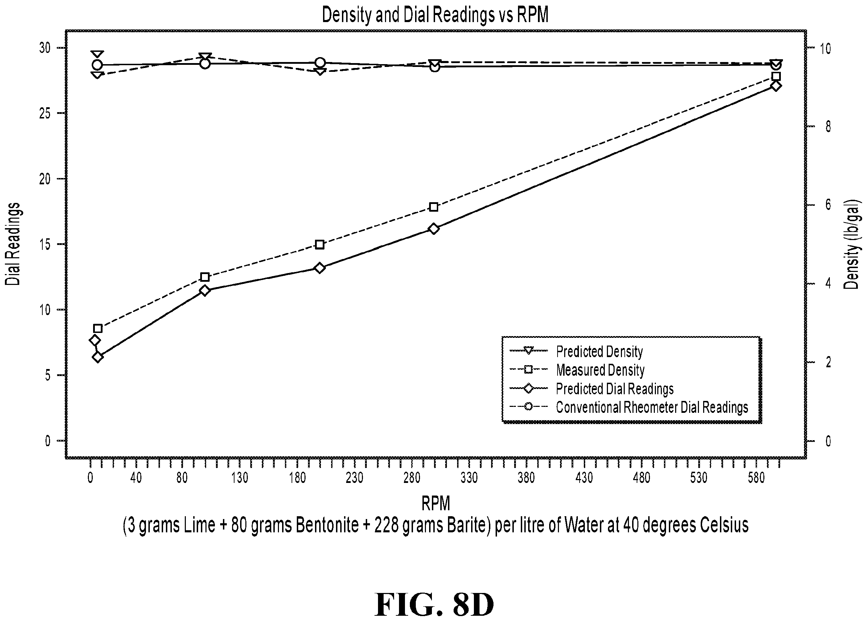

FIGS. 8A-8D show results from tests with fluids containing solids as is typical in the petroleum industry for drilling. FIG. 8A illustrates a solution of 3 grams of lime per liter of water. FIG. 8B illustrates a solution of 3 grams of lime and 40 grams of Bentonite per liter of water. FIG. 8C illustrates a solution of 3 grams of water and 80 grams of Bentonite per liter of water. FIG. 8D illustrates a solution of 3 grams of water, 80 grams of Bentonite, and 228 grams of Barite per liter of water. Water, lime, bentonite, and barite mixed in varying proportions are subjected to test on the flow loop. The acoustic sensing technique fared well in these tests on multiphase fluids. The discrepancies from the readings obtained on the conventional rheometers can be attributed to possible segregation of heavy solids in the fluid across various sections of the flow loop.

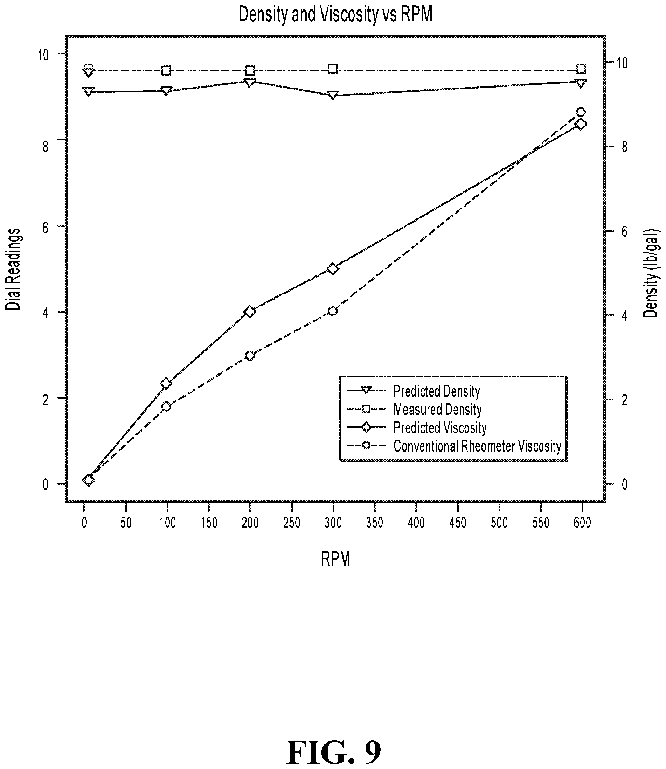

Finally, a setup and model was tested using an actual drilling fluid obtained from a rig which was drilling the intermediate-hole section in an Eagle Ford well. The results obtained were again compared with that from a conventional viscometer and a close match was obtained as shown in FIG. 9. In the petroleum industry, it is common to report the plastic viscosity (PV) and yield point (YP) derived from the 300 rpm and 600 rpm readings. A test was performed at 40°C (104°F) and the acoustic flow loop reported 300 rpm and 600 rpm readings of 5.0 and 8.34 (dial readings or D.R.), respectively; whereas the conventional rotational viscometer gave 4.0 and 8.6 dial readings respectively. This resulted in PV and YP values of 3.34 cP and 1.66 D.R., respectively, whereas the PV and YP for the conventional viscometer were 4.6 cP and -0.6 D.R., respectively. The mud was retested in the laboratory several times on the mud balance and rotational viscometer and all comparisons made in this example were based on the values obtained. The field testing conditions were different, at least in temperature, but perhaps in some other conditions too, and might have invariably resulted to some recorded discrepancies.

The density of the mud sample was 9.6 lb/gal as read from the mud balance. The 3, 200, and 600 rpm sensors had the most accuracy in the density measurements with values of 9.57, 9.33, and 9.29 lb/gal, respectively. The 300 rpm sensor showed the most discrepancy at 9.03 lb/gal. These variations may be attributed to a possible inhomogeneity in some sections of the flow loop, although the example set-up was designed to minimize such effect by vigorously stirring the fluids constantly. The viscosity measurements nonetheless were well within the range of accuracy of the conventional viscometer, with all measured dial readings from the acoustic method within ±1 D.R. of the rotational viscometer readings.

As such, the present disclosure shows that rheology and density can be measured in real-time and accurately via the proposed acoustic method. This is significantly different from the current state of the art in that it is non-intrusive and is continuous (i.e. no discrete samples needed). These characteristics make it capable of conducting a mud test at all standard shear rates continuously and simultaneously. Providing this capability to the oil industry would increase drilling performance and operational safety.

The inexpensive and easily installable acoustic apparatus offers great advantages in enabling real-time measurement of viscosity and density, which will yield additional advantages in field applications such as, instantaneous determination of Reynolds number and kinematic viscosity. Examples were demonstrated on a drilling mud used to drill wells in Eagle Ford, Texas and the readings got from the new method closely matched that from the conventional viscometer. Further testing on different mud systems is envisaged in the expectancy of applying the technique directly on a field location in the future.

The current configuration contains flow loop sections measuring one foot each, which resulted in non-fully developed flows during the operation of the flow loop. However, even under less than ideal conditions, good results were obtained. This indicates even better performance when operated in the field and at other applications where fully developed flows are attainable. The Multivariate Random Forest package in the statistical analysis and data analytics programming language "R" used for the interpretation of the results proved to be a reliable algorithm well suited for such a study requiring determination of new bivariate outcomes based upon prior learning on data. Calibrating the acoustic flow loop with a larger variety of fluid types, plus further tuning of the models' hyper-parameters, could help to improve the model, especially the viscosity which has a wider variation in different fluids studied. Additionally, alternative machine learning techniques such as XGBoost gradient boosting could be implemented in the future for comparison of performance, when such algorithms become adapted to the implementation of multi-task regression. By way of example, aspects of the disclosure are described herein as using the statistics/data analytics programming language "R"; however, in other embodiments, other programming languages such as, for example Python could also be utilized

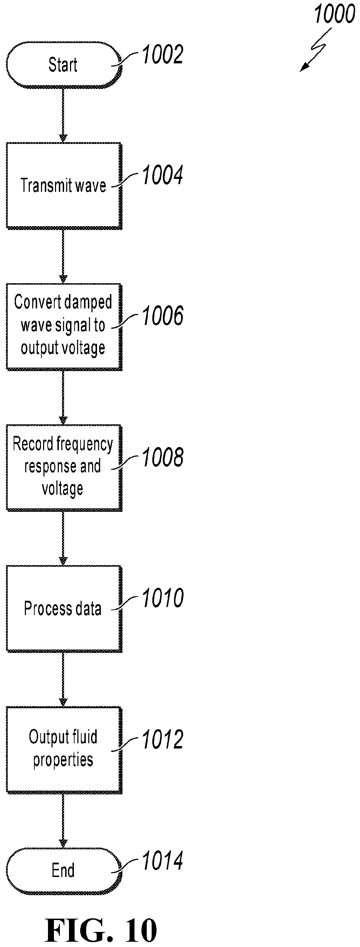

FIG. 10 illustrates a process 1000 for acoustic measurement of fluids. The process 1000 begins at step 1002. At step 1004, a wave is transmitted from the first sensor 104 through a fluid the second sensor 104. In various embodiments, the first sensor 104 is a first piezoelectric disc and the second sensor 106 is a second piezoelectric disc. At step 1006, the second sensor converts a damped wave signal into a voltage output by oscillating due to excitation. At step 1008, a data acquisition device records a frequency response and a voltage generated by the second sensor responsive to the damped wave signal. At step 1010, the data recorded by the data acquisition device is processed via a machine-learning algorithm.

At step 1012, fluid properties related to the fluid are output. The process 1000 ends at step 1014.

Applications and Advantages

In view of the above, in some embodiments, the present disclosure relates to systems with a series of piezoelectric sensors setup to measure fluid properties at different velocities. In some embodiments, each velocity represents a rheological data point, and is used for standard drilling fluid tests. In some embodiments, the systems of the present disclosure allow for continuous and constant volume flow, yet also allow for measurements of different conditions simultaneously.

In some embodiments, the systems as disclosed herein can be a series of pipes made to a particular size and length such that they are able to replicate fluid shear rates used in fluid characterization. The diameter can determine the equivalent shear rate, and the length is enough to allow for stable flow to develop. In some embodiments, the pipes have piezoelectric sensors mounted in such a way as to acquire data that will allow for the calculation of shear stress or what is commonly called "dial reading", while not disturbing the flow of the fluid. In some embodiments, the voltage inputs and outputs are sent in real-time to processing device, such as, for example, a computer that converts the values to dial readings at the commonly used shear rates. The systems as disclosed herein can include one or more pumps that are controlled to deliver the flow rates needed to flow fluid through the system such that the velocities, and thus the shear rates, are as required for proper measurements. In some embodiments, the pumps may be stand alone in the sense they are set at a given output and not varied, or the pump output may be controlled by the processing device and/or algorithm to vary and acquire multiple dial readings for single pipe geometries. The systems as disclosed herein can also be arranged to acquire gel strengths, or how a fluid behaves after a fixed period of rest.

The systems and methods, as disclosed herein, are envisioned as equipment on the surface of a wellsite or to a facility, and could also be deployed in a downhole environment on a logging while drilling tool. Furthermore, the systems as disclosed herein could also be deployed in downhole logging tool used for well testing or production testing purposes, as the concept work irrespective of overall size.

In some embodiments, the present disclosure relates to a method of using acoustic signals in a continuous, non-intrusive, automatable way to measure fluid properties. This is in contrast to the current state of the art where one has to take a sample, go through a series of measurements using a viscometer, which is not as accurate, and then report the values. In the time it takes to do this, the drilling fluid may have changed. The methods of the present disclosure work in any application where continual monitoring of drilling fluid properties is required. In some embodiments, this involves the acquisition of data from sensors, such as those discussed above, processing of data through machine learning algorithms, and the resulting output of density and shear stress (i.e. dial readings) at the shear rates measured. This output is further used to output various viscosities such as apparent viscosity and plastic viscosity, to name a few. In some embodiments, the methods presented herein would be part of a standard mud check if using drilling or completion fluids, or fluid characterization if used in other industries. For gel strength, the measurement is taken, for example, using the systems as described herein. In some embodiments, this provides a semi-continuous measurement, and due to the fact that very small pressure waves are used to measure this property, the methods herein yield a more realistic measurement of the fluid properties as a function of sitting still.

In some embodiments, the data is acquired from the systems as disclosed herein, or via another method. At this point it is only the input voltages and the frequency and range of how the piezo-device is activated. In some embodiments, the algorithm selects the type of input voltage based on the situation, primarily, but not limited to, a pulse of a single frequency or a sweep of a single frequency. The received response is recorded, checked for quality, and run through a machine learning algorithm to output the density and dial readings. There is some calibration or tuning of the algorithm required in development where a wide variety of fluid types are measured in the traditional manner and the proposed manner. However, in some embodiments, the algorithm can take inputs or spot checks from a traditional full mud check onsite to help tune the system setup in a new facility or wellsite, as the fluid type changes (or for any other perceived changes in fundamental baseline properties changes). The gel strength measurement process involves the fluid sitting still for a prescribed period of time, usually 1 () seconds, 10 minutes, 30 minutes, or at times up to an hour or more, After sitting still, the piezo-devices are activated and a measurements taken. The fluid remains at rest until the next time step and another measurement is taken. As with the previous methods, the algorithm uses trained machine learning algorithms to output gel strengths.

The field of fluid characterization has been relatively static for many years. In the drilling industry, the API mud check is still the standard and is woefully inadequate for the high risk, fast-paced oil and gas industry. In other industries, real-time fluid measurement generally involved invasive or in-line sensors, such as, for example, micro-motion-style sensors or approaches such as, for example, microfluidic devices that are unable to handle fluid containing solids. The systems and methods of the present disclosure are unique in that they do not put a physical restriction into the flow path of the fluid, they can be deployed onto, and are compatible with any process using fluids, whether oil and gas related, or other processes. Additionally, the systems and methods disclosed herein give a real-time, continuous, measurement of drilling fluid properties in a small, easy to deploy equipment package that requires a very low level of maintenance. Furthermore, the systems and methods, in some embodiments, can be used in dangerous environments or remote environments where the remote, real-time monitoring of fluid properties is preferred. The systems and methods disclosed herein use standard components, with the exception of the custom piping, to measure the properties, making maintenance and repair simple. Moreover, the systems and methods disclosed herein can handle very high levels of solids and particulate matter which other systems cannot.

Furthermore, the systems and methods of the present disclosure, in some embodiments, use piezoelectric generated waves or pulse data to calculate a wide range of fluid properties. In some embodiments, real-time, continuous, measurements of drilling fluid properties are possible, and additionally, the systems and methods of the present disclosure give a more accurate or true picture of the fluid properties. Moreover, the systems and methods presented herein have the ability to self-tune and/or calibrate using data that is already gathered at the wellsite or the facility. Additionally, what has previously been thought of as a useless and often misleading piece of equipment that should be removed from drilling rigs can become a quite valuable tool. The methods disclosed herein allow the user to input a single number into either the graphical user interface of the systems disclosed herein, an application, calculator, or an EDR system on the rig, and instantly get a synthetic set of dial readings, allowing for quick analysis of the fluid or if used in conjunction with the systems disclosed herein, allow for the calibration of such systems.

Although various embodiments of the present disclosure have been illustrated in the accompanying Drawings and described in the foregoing Detailed Description, it will be understood that the present disclosure is not limited to the embodiments disclosed herein, but is capable of numerous rearrangements, and modifications.

The term "substantially" is defined as largely but not necessarily wholly what is specified, as understood by a person of ordinary skill in the art. In any disclosed embodiment, the terms "substantially", "approximately", "generally", and "about" may be substituted with "within [a percentage] of" what is specified, where the percentage includes 0.1, 1, 5, and 10 percent.

The foregoing outlines features of several embodiments so that those skilled in the art may better understand the aspects of the disclosure. Those skilled in the art should appreciate that they may readily use the disclosure as a basis for designing or modifying other processes and structures for carrying out the same purposes and/or achieving the same advantages of the embodiments introduced herein. The scope of the invention should be determined only by the language of the claims that follow. The term "comprising" within the claims is intended to mean "including at least" such that the recited listing of elements in a claim are an open group. The terms "a", "an", and other singular terms are intended to include the plural forms thereof unless specifically excluded.

**Claims**

1. A system for monitoring fluid properties in real-time, the system comprising:
   a flow loop (600) having a fluid inlet and a fluid outlet, the flow loop (600) comprising:

   a plurality of pipe sections (602), (604), (606), (608), (610), (612) forming the flow loop (600), wherein each pipe section of the plurality of pipe sections (602), (604), (606), (608), (610), (612) is in fluid communication with each other; and sensor pairs, the sensors being
   piezoelectric devices (614), (616), (618), (620), (622), (624) fluidly coupled to each of the pipe sections perpendicular to fluid flow within each of the pipe sections;
   **characterized in that**
   each of the pipe sections comprises an inner diameter that differs from each other pipe section;
   wherein each of the pipe sections of the plurality of pipe sections (602), (604), (606), (608), (610), (612) comprise a size and length such that each of the pipe sections replicate pre-determined fluid shear rates; and
   wherein the piezoelectric devices (614), (616), (618), (620), (622), (624) measure fluid at different velocities corresponding to the differing inner diameters of each of the pipe sections.

2. The system of claim 1, comprising a wave generator electrically coupled to the piezoelectric devices (614), (616), (618), (620), (622), (624).

3. The system of claim 1, wherein the fluid flow is maintained at a continuous and constant volume flow.

4. The system of claim 1, wherein the piezoelectric devices (614), (616), (618), (620), (622), (624) are piezoelectric discs and utilize acoustic signals to measure fluid properties.

5. The system claim 1, comprising six pipe sections (602), (604), (606), (608), (610), (612) of six different pipe diameters.

6. The system of claim 5, wherein the six pipe sections (602), (604), (606), (608), (610), (612) are configured to replicate the following six pre-determined fluid shear rates: 5.11 s$^{-1}$, 10.21 s$^{-1}$, 170.23 s$^{-1}$, 340.46 s$^{-1}$, 510.69 s$^{-1}$, and 1021.38 s$^{-1}$.

7. The system of claim 5 or claim 6, comprising six sensor pairs placed at each pipe section to measure the fluid properties at six pre-determined fluid shear rates.

8. The system of any one of claims 1 to 7, wherein a first piezoelectric device and a second piezoelectric device of the piezoelectric devices (614), (616), (618), (620), (622), (624) are arranged parallel to each other, and wherein the first piezoelectric device is disposed on a first side of the flow loop (600) and the second piezoelectric device is disposed on a second side of the flow loop (600) opposite the first side.

9. A method for monitoring fluid properties in real-time, the method comprising:

> passing a fluid through a flow loop (600) having a fluid inlet and a fluid outlet, the flow loop (600) comprising:

>> a plurality of pipe sections (602), (604), (606), (608), (610), (612) forming the flow loop (600), wherein each pipe section of the plurality of pipe sections (602), (604), (606), (608), (610), (612) is in fluid communication with each other; and sensor pairs, the sensors being
>> piezoelectric devices (614), (616), (618), (620), (622), (624) fluidly coupled to each of the pipe sections perpendicular to fluid flow within each of the pipe sections;
>> recording data, by a data acquisition system, sensed by the piezoelectric devices; and
>> processing the recorded data to determine fluid properties related to the fluid;

> wherein each of the pipe sections comprise an inner diameter that differs from each other pipe section, the inner diameters corresponding to different pre-determined shear rates; and
> wherein the piezoelectric devices (614), (616), (618), (620), (622), (624) measure fluid at different velocities corresponding to the differing inner diameters of each of the pipe sections.

10. The method of claim 9, wherein the recorded data is processed via a machine learning algorithm.

11. The method of claim 9 or claim 10, wherein the fluid properties are at least one of density, shear stress, viscosity, and gel strength of the fluid.

12. The method of any one of claims 9-11, further comprising determining at least one of apparent viscosity and plastic viscosity from the fluid properties.

13. The method of any one of claims 9-12, wherein the fluid is a drilling fluid.

14. The method of claim 13, wherein the drilling fluid is passed through a flow loop (600) comprising six pipe sections (602), (604), (606), (608), (610), (612) of six different pipe diameters.

**Patentansprüche**

1. System zum Überwachen von Fluideigenschaften in Echtzeit, das System umfassend:
einen Strömungskreislauf (600) mit einem Fluideinlass und einem Fluidauslass, wobei der Strömungskreislauf (600) Folgendes umfasst:

> eine Vielzahl von Rohrabschnitten (602), (604), (606), (608), (610), (612), die den Strömungskreislauf (600) bilden, wobei jeder Rohrabschnitt der Vielzahl von Rohrabschnitten (602), (604), (606), (608), (610), (612) in Fluidverbindung miteinander steht; und Sensorpaare, wobei die Sensoren piezoelektrische Vorrichtungen (614), (616), (618), (620), (622), (624), die senkrecht zu der Fluidströmung innerhalb jedes der Rohrabschnitte fluidisch an jeden der Rohrabschnitte gekoppelt sind;
> **dadurch gekennzeichnet, dass**
> jeder der Rohrabschnitte einen Innendurchmesser umfasst, der sich von jedem anderen Rohrabschnitt unterscheidet;
> wobei jeder der Rohrabschnitte der Vielzahl von Rohrabschnitten (602), (604), (606), (608), (610), (612) eine solche Größe und Länge umfasst, dass jeder der Rohrabschnitte vorbestimmte Fluidscherraten reproduziert; und

wobei die piezoelektrischen Vorrichtungen (614), (616), (618), (620), (622), (624) Fluid bei unterschiedlichen Geschwindigkeiten messen, die den unterschiedlichen Innendurchmessern jedes der Rohrabschnitte entsprechen.

2. System nach Anspruch 1, umfassend einen Wellengenerator, der elektrisch an die piezoelektrischen Vorrichtungen (614), (616), (618), (620), (622), (624) gekoppelt ist.

3. System nach Anspruch 1, wobei die Fluidströmung auf einer kontinuierlichen und konstanten Volumenströmung gehalten wird.

4. System nach Anspruch 1, wobei die piezoelektrischen Vorrichtungen (614), (616), (618), (620), (622), (624) piezoelektrische Scheiben sind und akustische Signale nutzen, um Fluideigenschaften zu messen.

5. System nach Anspruch 1, umfassend sechs Rohrabschnitte (602), (604), (606), (608), (610), (612) mit sechs unterschiedlichen Rohrdurchmessern.

6. System nach Anspruch 5, wobei die sechs Rohrabschnitte (602), (604), (606), (608), (610), (612) dazu konfiguriert sind, die folgenden sechs vorbestimmten Fluidscherraten zu reproduzieren: $5,11 \text{ s}^{-1}$, $10,21 \text{ s}^{-1}$, $170,23 \text{ s}^{-1}$, $340,46 \text{ s}^{-1}$, $510,69 \text{ s}^{-1}$ und $1021,38 \text{ s}^{-1}$.

7. System nach Anspruch 5 oder Anspruch 6, umfassend sechs Sensorpaare, die an jedem Rohrabschnitt platziert sind, um die Fluideigenschaften bei sechs vorbestimmten Fluidscherraten zu messen.

8. System nach einem der Ansprüche 1 bis 7, wobei eine erste piezoelektrische Vorrichtung und eine zweite piezoelektrische Vorrichtung der piezoelektrischen Vorrichtungen (614), (616), (618), (620), (622), (624) parallel zueinander angeordnet sind und wobei die erste piezoelektrische Vorrichtung auf einer ersten Seite des Strömungskreislaufs (600) angeordnet ist und die zweite piezoelektrische Vorrichtung auf einer der ersten Seite gegenüberliegenden zweiten Seite des Strömungskreislaufs (600) angeordnet ist.

9. Verfahren zum Überwachen von Fluideigenschaften in Echtzeit, wobei das Verfahren Folgendes umfasst:
Leiten eines Fluid durch einen Strömungskreislauf (600) mit einem Fluideinlass und einem Fluidauslass, wobei der Strömungskreislauf (600) Folgendes umfasst:

eine Vielzahl von Rohrabschnitten (602), (604), (606), (608), (610), (612), die den Strömungskreislauf (600) bilden, wobei jeder Rohrabschnitt der Vielzahl von Rohrabschnitten (602), (604), (606), (608), (610), (612) in Fluidverbindung miteinander steht; und Sensorpaare, wobei die Sensoren piezoelektrische Vorrichtungen (614), (616), (618), (620), (622), (624), die senkrecht zu der Fluidströmung innerhalb jedes der Rohrabschnitte fluidisch an jeden der Rohrabschnitte gekoppelt sind;
Aufzeichnen von Daten, die durch die piezoelektrischen Vorrichtungen erfasst werden, durch ein Datenerfassungssystem; und
Verarbeiten der aufgezeichneten Daten, um Fluideigenschaften in Bezug auf das Fluid zu bestimmen;
wobei jeder der Rohrabschnitte einen Innendurchmesser umfasst, der sich von jedem anderen Rohrabschnitt unterscheidet, wobei die Innendurchmesser unterschiedlichen vorbestimmten Scherraten entsprechen; und
wobei die piezoelektrischen Vorrichtungen (614), (616), (618), (620), (622), (624) Fluid bei unterschiedlichen Geschwindigkeiten messen, die den unterschiedlichen Innendurchmessern jedes der Rohrabschnitte entsprechen.

10. Verfahren nach Anspruch 9, wobei die aufgezeichneten Daten mittels eines Algorithmus des maschinellen Lernens verarbeitet werden.

11. Verfahren nach Anspruch 9 oder Anspruch 10, wobei es sich bei den Fluideigenschaften um mindestens eines von einer Dichte, einer Scherspannung, einer Viskosität und einer Gelstärke des Fluids handelt.

12. Verfahren nach einem der Ansprüche 9-11, ferner umfassend Bestimmen von mindestens einer von einer scheinbaren Viskosität und einer plastischen Viskosität aus den Fluideigenschaften.

**13.** Verfahren nach einem der Ansprüche 9-12, wobei das Fluid ein Bohrfluid ist.

**14.** Verfahren nach Anspruch 13, wobei das Bohrfluid durch einen Strömungskreislauf (600) geleitet wird, der sechs Rohrabschnitte (602), (604), (606), (608), (610), (612) mit sechs unterschiedlichen Rohrdurchmessern umfasst.

**Revendications**

**1.** Système de surveillance de propriétés de fluide en temps réel, le système comprenant :
une boucle d'écoulement (600) comportant une entrée de fluide et une sortie de fluide, la boucle d'écoulement (600) comprenant :

une pluralité de sections de tuyau (602), (604), (606), (608), (610), (612) formant la boucle d'écoulement (600), dans lequel chaque section de tuyau de la pluralité de sections de tuyau (602), (604), (606), (608), (610), (612) est en communication fluidique avec les autres ; et des paires de capteurs, les capteurs étant des dispositifs piézoélectriques (614), (616), (618), (620), (622), (624) couplés de manière fluidique à chacune des sections de tuyau perpendiculairement à l'écoulement de fluide à l'intérieur de chacune des sections de tuyau ;
**caractérisé en ce que**
chacune des sections de tuyau comprend un diamètre intérieur qui diffère de chaque autre section de tuyau ;
dans lequel chacune des sections de tuyau de la pluralité de sections de tuyau (602), (604), (606), (608), (610), (612) comprend une taille et une longueur de sorte que chacune des sections de tuyau reproduit des taux de cisaillement de fluide prédéterminés ; et
dans lequel les dispositifs piézoélectriques (614), (616), (618), (620), (622), (624) mesurent du fluide à différentes vitesses correspondant aux différents diamètres intérieurs de chacune des sections de tuyau.

**2.** Système de la revendication 1, comprenant un générateur d'ondes couplé électriquement aux dispositifs piézoélectriques (614), (616), (618), (620), (622), (624).

**3.** Système de la revendication 1, dans lequel l'écoulement de fluide est maintenu à un écoulement volumique continu et constant.

**4.** Système de la revendication 1, dans lequel les dispositifs piézoélectriques (614), (616), (618), (620), (622), (624) sont des disques piézoélectriques et utilisent des signaux acoustiques pour mesurer des propriétés de fluide.

**5.** Système de la revendication 1, comprenant six sections de tuyau (602), (604), (606), (608), (610), (612) de six diamètres de tuyaux différents.

**6.** Système de la revendication 5, dans lequel les six sections de tuyau (602), (604), (606), (608), (610), (612) sont conçues pour reproduire les six taux de cisaillement de fluide prédéterminés suivants : $5,11 \text{ s}^{-1}$, $10,21 \text{ s}^{-1}$, $170,23 \text{ s}^{-1}$, $340,46 \text{ s}^{-1}$, $510,69 \text{ s}^{-1}$ et $1021,38 \text{ s}^{-1}$.

**7.** Système de la revendication 5 ou de la revendication 6, comprenant six paires de capteurs placées au niveau de chaque section de tuyau pour mesurer des propriétés du fluide à six taux de cisaillement de fluide prédéterminés.

**8.** Système de l'une quelconque des revendications 1 à 7, dans lequel un premier dispositif piézoélectrique et un second dispositif piézoélectrique des dispositifs piézoélectriques (614), (616), (618), (620), (622), (624) sont agencés parallèlement l'un à l'autre, et dans lequel le premier dispositif piézoélectrique est disposé sur un premier côté de la boucle d'écoulement (600) et le second dispositif piézoélectrique est disposé sur un second côté de la boucle d'écoulement (600) opposé au premier côté.

**9.** Procédé de surveillance de propriétés de fluides en temps réel, le procédé comprenant :
le passage d'un fluide à travers une boucle d'écoulement (600) comportant une entrée de fluide et une sortie de fluide, la boucle d'écoulement (600) comprenant :

une pluralité de sections de tuyau (602), (604), (606), (608), (610), (612) formant la boucle d'écoulement (600), dans lequel chaque section de tuyau de la pluralité de sections de tuyau (602), (604), (606), (608), (610), (612) est en communication fluidique avec les autres ; et des paires de capteurs, les capteurs étant des dispositifs piézoélectriques (614), (616), (618), (620), (622), (624) couplés de manière fluidique à chacune des sections de tuyau perpendiculairement à l'écoulement de fluide à l'intérieur de chacune des sections de tuyau ;
l'enregistrement de données, par un système d'acquisition de données, détectées par les dispositifs piézoélectriques ; et
le traitement des données enregistrées pour déterminer des propriétés de fluide en lien avec le fluide ;
dans lequel chacune des sections de tuyau comprend un diamètre intérieur qui diffère de chaque autre section de tuyau, les diamètres intérieurs correspondant à différents taux de cisaillement prédéterminés ; et
dans lequel les dispositifs piézoélectriques (614), (616), (618), (620), (622), (624) mesurent du fluide à différentes vitesses correspondant aux différents diamètres intérieurs de chacune des sections de tuyau.

10. Procédé de la revendication 9, dans lequel les données enregistrées sont traitées par l'intermédiaire d'un algorithme d'apprentissage automatique.

11. Procédé de la revendication 9 ou la revendication 10, dans lequel les propriétés du fluide sont au moins l'une d'une densité, d'une contrainte de cisaillement, d'une viscosité et d'une résistance de gel du fluide.

12. Procédé de l'une quelconque des revendications 9 à 11, comprenant en outre
la détermination d'au moins l'une d'une viscosité apparente et d'une viscosité plastique à partir des propriétés de fluide.

13. Procédé de l'une quelconque des revendications 9 à 12, dans lequel le fluide est un fluide de forage.

14. Procédé de la revendication 13, dans lequel le fluide de forage passe à travers une boucle d'écoulement (600) comprenant six sections de tuyau (602), (604), (606), (608), (610), (612) de six diamètres de tuyau différents.

FIG. 1

FIG. 2B

10.21 (1/seconds) sensor

Relative Amplitude (db)

Frequency (Hz)

FIG. 2A

5.11 (1/seconds) sensor

Relative Amplitude (db)

Frequency (Hz)

FIG. 2C

FIG. 2D

EP 4 028 747 B1

510.69 (1/seconds) sensor

**FIG. 2E**

1021.38 (1/seconds) sensor

**FIG. 2F**

Density and Viscosity Readings vs Shear Rate

0.5 gram per litre Methyl cellulose solution at 20 degrees Celsius

**FIG. 3A**

Density and Viscosity Readings vs Shear Rate

0.5 gram per litre Methyl cellulose solution at 20 degrees Celsius

**FIG. 3B**

Density and Viscosity Readings vs Shear Rate

0.5 gram per litre Methyl cellulose solution at 20 degrees Celsius

**FIG. 4A**

EP 4 028 747 B1

Density and Viscosity Readings vs Shear Rate

0.5 gram per litre Calcium Chloride solution at 40 degrees Celsius

**FIG. 4B**

FIG. 5

**FIG. 6**

**Density and Dial Readings vs RPM**

Saturated Calcium Chloride solution at 40degrees Celsius

Legend:
- Predicted Density
- Measured Density
- Predicted Viscosity
- Conventional Rheometer Viscosity

**FIG. 7A**

**FIG. 7B**

FIG. 7C

FIG. 7D

EP 4 028 747 B1

Density and Dial Readings vs RPM

Lime solution: 3 grams per litre of Water at 40 degrees Celsius

**FIG. 8A**

Density and Dial Readings vs RPM

(3 grams Lime + 40 grams Bentonite) per litre of Water at 40 degrees Celsius

**FIG. 8B**

EP 4 028 747 B1

FIG. 8C

EP 4 028 747 B1

Density and Dial Readings vs RPM

(3 grams Lime + 80 grams Bentonite + 228 grams Barite) per litre of Water at 40 degrees Celsius

**FIG. 8D**

EP 4 028 747 B1

FIG. 9

FIG. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62897662
- US 5437194 A
- US 7290441 B2
- US 2019178770 A1
- WO 2018175503 A2